(19) Europäisches Patentamt
European Patent Office
Office européen des brevets

(11) Veröffentlichungsnummer: **0 191 792 B1**

(12) # EUROPÄISCHE PATENTSCHRIFT

(45) Veröffentlichungstag der Patentschrift: **22.01.92**

(51) Int. Cl.5: **A61K 31/557**, A61K 31/34, A61K 31/275

(21) Anmeldenummer: **85903809.3**

(22) Anmeldetag: **18.07.85**

(86) Internationale Anmeldenummer:
**PCT/DE85/00245**

(87) Internationale Veröffentlichungsnummer:
**WO 86/00808 (13.02.86 86/04)**

(54) **PROSTACYCLIN-DERIVATE MIT ZYTOPROTEKTIVER WIRKUNG AN DER NIERE.**

(30) Priorität: **25.07.84 DE 3427797**

(43) Veröffentlichungstag der Anmeldung:
**27.08.86 Patentblatt 86/35**

(45) Bekanntmachung des Hinweises auf die Patenterteilung:
**22.01.92 Patentblatt 92/04**

(84) Benannte Vertragsstaaten:
**AT BE CH DE FR GB IT LI LU NL SE**

(56) Entgegenhaltungen:
**EP-A- 0 002 234**
**EP-A- 0 011 591**
**DE-A- 3 318 571**

**CHEMICAL ABSTRACTS; Band 99, Nr. 15, 10. Oktober 1983, Columbus, Ohio, USA; O. SIKUJARA et al: "Cytoprotective effect of prostaglandin I2 on ischemia-induced hepatic cell injury", siehe S. 161,162, Zusammenfassung 116850a**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **ADLER, Guido**
**Landgrafenweg 7**
**W-3550 Marburg(DE)**
Erfinder: **SCHULTE-HERMANN, Rolf**
**Freiherr-vom-Stein-Str. 30**
**W-3550 Marburg 6(DE)**
Erfinder: **LOGE, Olaf**
**Bekassinenweg 37**
**W-1000 Berlin 27(DE)**

**EP 0 191 792 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICAL ABSTRACTS, Band 99, Nr. 3, 18. Juli 1983, Columbus, Ohio, USA; M. MONDEN et al: "Cytoprotective effect of prostaglandin I2 on ischemia-induced hepatic cell injury", siehe S. 151, Zusammenfassung 17252a**

**CHEMICAL ABSTRACTS, Band 92, Nr. 15, 4, April 1980, Columbus, Ohio, USA; H. ARAKI et al: "Cytoprotective actions of prostacyclin during hypoxia in the isolated perfused cat liver", siehe S. 84, Zusammenfassung 122181e**

**Beschreibung**

Die vorliegende Erfindung betrifft ein Mittel mit zytoprotektiver Wirkung an der Niere, das als Wirkstoff Iloprost oder Nileprost enthält, sowie ein Verfahren zur Herstellung dieser Mittel.

Aus EP 11 591 ist bereits die zytoprotektive Wirkung von Carbacyclinderivaten an der Magen- und Darmschleimhaut sowie die myocardiale Zytoprotektion mit Hilfe von Carbacyclinderivaten bekannt.

Es wurde nun gefunden, daß Iloprost (I) und Nileprost (II)

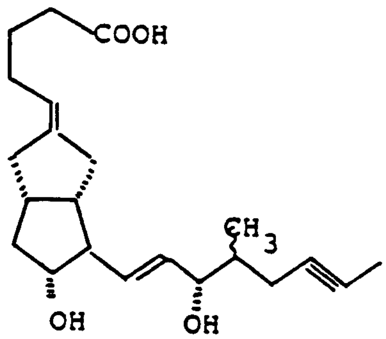

(I)

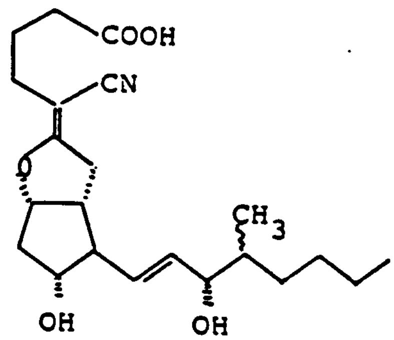

(II)

und deren Salze mit physiologisch verträglichen Basen auch in der Niere zytoprotektiv wirken.

Zur Salzbildung von Iloprost und Nileprost sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Herstellung von Iloprost und Nileprost wird detailliert in EP 2234 und EP 11591 beschrieben.

In EP 11591 werden für die dort beschriebenen Carbacyclinderivate folgende pharmakologische Eigenschaften beschrieben:

Senkung des peripheren arteriellen und koronaren vaskulären Widerstandes, Inhibierung der Thrombozytenaggregation und Auflösung von Plättchenthromben, myocardiale Zytoprotektion; Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion und Zytoprotektion der Magen- und Darmschleimhaut; antiallergische Eigenschaften, Senkung des pulmonalen vaskulären Widerstandes und des pulmonalen Blutdruckes, Förderung der Nierendurchblutung, Anwendung an Stelle von Heparin oder als Adjuvans bei der Dialyse oder Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung und Antiproliferation.

Die Zytoprotektion der Niere wird nicht beschrieben und steht auch in keinem direkten Zusammenhang mit den in EP 11591 beschriebenen Zytoprotektionen. Iloprost und Nileprost eignen sich außerdem zur Behandlung von zu transplantierenden Organen.

In der Niere wirken Iloprost und Nileprost überraschend zytoprotektiv. Für die Niere ließ sich der cytoprotektive Effekt als Schutzwirkung gegenüber der Induktion von Papillennekrosen durch nichtsteroidale Entzündungshemmstoffe nachweisen.

Die Dosis beider Verbindungen ist 1-1500µg/kg/Tag, wenn sie am menschlichen Patienten verabreicht werden. Die Einheitsdosis für den pharmazeutischen akzeptablen Träger beträgt 0,01-100 mg.

Die Dosierung einer i.v.-Verabreichung als Dauerinfusion in üblichen wässrigen Lösungsmitteln, z.B. 0,9%iger NaCl-Lösung, erfolgt vorzugsweise in Dosierungen zwischen 0,1 ng/kg/min und 0,1 µg/kg/min.

Die Erfindung betrifft damit auch Arzneimittel auf Basis der genannten Verbindungen und üblicher Hilfs- und Trägerstoffe.

Die erfindungsgemäßen Wirkstoffe sollen in Verbindung mit den in der Galenik bekannten und üblichen Hilfsstoffen z.B. zur Herstellung von Zytoprotektiva dienen.

Die Erfindung betrifft auch ein Verfahren zur Herstellung der erfindungsgemäßen Mittel, das dadurch gekennzeichnet ist, daß man in an sich bekannter Weise die zytoprotektiv wirkenden Verbindungen mit den

an sich bekannten Hilfs- und Trägerstoffen in eine galenische Formulierung bringt.

Beispiel 1

Galenische Zubereitung

| | |
|---|---|
| 0,5 mg | 5-{(E)-(1S,5S,6R,7R)-7-Hydroxy-6-[(E)-(4RS)-3α-hydroxy-4-methyl-oct-1-en-6-inyl]-bicyclo [3.3.0]octan-3-yliden}-pentansäure |
| 1,212 mg | Tromethanol-Puffer |
| ad pH 8,3 | 0,1 n Salzsäure |
| 8,9 mg | NaCl |
| 0,01 ml | 96%iges Äthanol |
| ad 1,0 ml | für Injektionszwecke |

## Patentansprüche

**1.** Verwendung von Iloprost zur Herstellung eines Mittels zur Behandlung oder Verhinderung von Schädigungen der Niere.

**2.** Verwendung von Nileprost zur Herstellung eines Mittels zur Behandlung oder Verhinderung von Schädigungen der Niere.

## Claims

**1.** Use of iloprost for the preparation of an agent for the treatment or prevention of damage to the kidney.

**2.** Use of nileprost for the preparation of an agent for the treatment or prevention of damage to the kidney.

## Revendications

**1.** Utilisation d'Iloprost pour la préparation d'un produit pour le traitement ou l'empêchement d'endommagements aux reins.

**2.** Utilisation de Nileprost pour la préparation d'un produit pour le traitement ou l'empêchement d'endommagements aux reins.